# EUROPEAN PATENT SPECIFICATION

(11) **EP 4 233 836 B1**
(45) Date of publication and mention of the grant of the patent: **06.11.2024**
(21) Application number: 22158449.3
(22) Date of filing: 24.02.2022
(51) Int. Cl.: A61K 9/00, A61F 9/00

(54) **INTRAOCULAR DRUG DELIVERY DEVICE COMPRISING AN EYELET**
INTRAOKULARE ARZNEIMITTELABGABEVORRICHTUNG, DIE EINE ÖSE UMFASST
DISPOSITIF D'ADMINISTRATION INTRAOCULAIRE DE MEDICAMENT COMPRENANT UN OEILLET

(43) Date of publication of application: 30.08.2023
(73) Proprietor: Eyed Pharma, 4102 Seraing (BE)
(72) Inventor: ALAIMO, David, 4550 Nandrin (BE); WIJZEN, Fabienne, 4140 Sprimont (BE); NIZET, Dominique, 4020 Liège (BE)
(74) Representative: Brantsandpatents bv

(56) References cited:
- WO-A1-2014/066775
- WO-A1-2015/071427
- US-A1- 2013 302 398

## Description

### TECHNICAL FIELD

The present invention as defined in the appended claims relates to a sustained release drug delivery device/implant, specifically, a removable sustained release drug delivery device/implant intended for the prolonged and controlled release of one or more therapeutic agent, and more particularly, a sustained release drug delivery device/implant configured to benefit the removal procedure.

### BACKGROUND

A variety of ophthalmic and non-ophthalmic conditions necessitate administration of various drugs to the eye. Eye drops and gels can be effective drug delivery vehicles, but can also have significant disadvantages. Specifically, eye drops mix with fluid in the tear film, but may have a residence time of only 2-5 minutes in the tear film. As little as 5% of the drug may be absorbed locally; some or all of the rest being carried from the lacrimal sac into the lacrimal duct and eventually absorbed into the bloodstream. The absorption into the bloodstream can have at least two adverse effects: first, most of the drug is wasted and, second, the presence of the drug in the bloodstream may have harmful side effects on the rest of the body. Gels may adhere more effectively to the eye, but can also blur the patient's vision for prolonged time periods. Both eye drops and gels need to be reapplied frequently for some therapies. Thus, a need remains for an improved drug delivery method to the eye that is neither cleared out of its targeted location, nor needs frequent administration.

In light of the disadvantages of eye drops, it is understandable that a variety of alternatives have been proposed. Among the known alternatives to drops include treatments in which structures containing or impregnated with drugs have been placed under the eyelid. Such solid ocular dosage forms appear to present significant potential advantages over drop-administered drug treatments of the eyes. In particular, eye drug delivery implants might help overcome low patient compliance, the difficult application and frequent misapplication of traditional eye drops and other dosage forms, and limited effective drug absorption presented by eye drops, while potentially facilitating the advantageous application of advances in polymer chemistry and introduction of the concepts of sustained/controlled drug release from other known drug-delivery devices/implants.

As an approach for circumventing the drawbacks encountered by local topical delivery, local therapy routes for the eye involving direct intravitreal injection of a drug through the sclera (i.e. the spherical, collagen-rich outer covering of the eye) have been tried. However, the intravitreal injection delivery route tends to result in a short half-life and rapid clearance of the drug, without sustained release capability being attained. Consequently, monthly injections are frequently required to maintain therapeutic ocular drug levels which is not practical for many patients, particularly in the treatment of degenerative or persistent conditions. Moreover, monthly intravitreal injections are not without risk, as some undesired effects associated with repeated injections can occur including sub-conjunctival hemorrhage, intraocular infections, worsening of pre-existing cataracts, accidental lesion to the lens, and/or retinal detachment.

Implantable intraocular sustained-release delivery devices/implants have the potential to avoid the shortcomings and complications that can arise from both systemic and local therapies (i.e. topical administration or intravitreal injections). However, despite the variety of ocular implant devices/implants which have been described and used in the art, the full potential of this therapy route has not been reached.

In view of the above, there remains a need in the art for improved non-degradable sustained release intraocular drug delivery devices, which are simple to manufacture, and capable of releasing a therapeutic agent at a sustained and controlled rate for extended periods of time, while allowing easy removal. We note that some biodegradable implants exist, but these typically last less than a year before fully degrading, which is undesirable.

The implantation of such a device is often assisted by an injecting device. US2019083313 A1 and US2019091012 A1 disclose typical devices and methods for controlling placement of intraocular implants within a patient's eye. The withdrawal of such a device is often achieved by the same injecting device, which removes the drug delivery device by picking up the body with a for instance, hook-shaped picking-up structure at the end of the injecting device.

EP3068372 B1 discloses a sustained release intraocular drug delivery device comprising: (a) a polymeric matrix core into which at least one therapeutic agent is mixed, and; (b) a polymeric coating completely surrounding said polymeric matrix material; wherein the degree of cross-linking in the polymer selected for the polymeric coating regulates the drug release rates from the intraocular drug device. In particular embodiments, the drug delivery device comprises a tag or protrusion to ease its manipulation during insertion or removal of said device. However, it is not explicitly disclosed how the protrusion helps the insertion or removal process. The technical feature of said protrusion remains unclear.

WO2010141729 A1 discloses a therapeutic system comprises an ocular insert placed on a region outside an optical zone of an eye. The ocular insert comprises two structures: a first skeletal structure and a second cushioning structure. However, the device does not generate a sustained release of a therapeutic agent. The first structure requires the second structure to be ring shape, and does not assist the withdrawal of the device.

One of the current goals in designing a non-degradable sustained intraocular drug delivery device is that it shall i) allow easy withdrawal of said device; ii) does not obstruct the implantation of the drug delivery device; iii) be simple to manufacture taking into account the complexity of manufacturing of components at micro-size.

The applicant notes that the above issues are especially problematic when the device itself is thinner, and thus more fragile. A thinner device is desirable, as it causes much less hinder for the patient (both in reduction of line of sight as well as in inconveniencing the eye) and can deliver the therapeutic agent more efficiently.

A further issue is that of linearization, i.e. allowing the device to be folded for placement in a compact (very narrow) configuration through a simple surgery, which is also addressed by the invention at hand.

Thus there is a need to design a specific feature in the sustained intraocular drug delivery device to solve one or more above addressed problems and, to be noted, such feature shall not cause further problems such as affecting the sustained release, bringing unpleasant user experience.

### SUMMARY OF THE INVENTION

Accordingly, it is the object of the present application to provide an improved sustained release intraocular drug delivery device, which solves at least some of the problems mentioned above.

The present inventors have designed an insoluble and inert sustained release intraocular drug delivery device, for insertion in the posterior chamber (or more specifically in the sulcus) of the eye. The insertion of the device in the sulcus of the eye does not initiate fibrotic processes and, due to its inert nature in ocular fluids, the device can be removed after it has been depleted from its drug or earlier, in case of unwanted side-effect to the drug or in-line to a determined scheme. Moreover, using the sulcus of the eye as insertion site allows the dimensions of the device to be such that sustained release of the therapeutic agent of choice can be attained for up to five years. Also, the invention contains an eyelet configured for assisting the withdrawal of the device. In addition, the inventors have discerned a simple method of manufacture of said intraocular device, allowing attaining product uniformity by standard manufacturing techniques.

The invention in a first aspect provides a sustained release intraocular drug delivery device, comprising: an elongate body, two (sealed) ends, an eyelet fixedly connected with at least one end,
wherein said elongate body further comprises a polymeric matrix core into which at least one therapeutic agent is mixed, and a polymeric coating completely surrounding said polymeric matrix material, wherein said eyelet is configured to assist the withdrawal of said device and wherein said eyelet is essentially free of the (and any other) therapeutic agent.

The inventors have thus realized a sustained release intraocular drug delivery device comprising an eyelet which is configured to assist the withdrawal of said device.

### DESCRIPTION OF FIGURES

**Figure 1A** shows schematically a perspective view of the sustained release intraocular drug delivery device according to an embodiment of the present application, with a first preferred embodiment of the eyelet.
**Figure 1B** shows schematically a perspective view of the dimensional details of an embodiment of Figure 1A.
**Figure 2A** shows schematically a perspective view of the sustained release intraocular drug delivery device according to an embodiment of the present application, with a second preferred embodiment of the eyelet.
**Figure 2B** shows schematically a perspective view of the dimensional details of an embodiment of Figure 2A.
**Figure 3A** shows schematically a perspective view of the sustained release intraocular drug delivery device according to an embodiment of the present application, with a third preferred embodiment of the eyelet.
**Figure 3B** shows schematically a perspective view of the dimensional details of an embodiment of Figure 3A.

### DETAILED DESCRIPTION OF THE INVENTION

Unless defined otherwise, all technical and scientific terms used herein have the same meaning as commonly understood by one of ordinary skill in the art to which this application belongs.

As used herein, the singular forms "a", "an", and "the" include both singular and plural referents unless the context clearly dictates otherwise. The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

The recitation of numerical ranges by endpoints includes all numbers and fractions subsumed within the respective ranges, as well as the recited endpoints. When a numerical value is used, said value encompasses the exact numerical value as such, as well as all numerical values that would be rounded up to said exact numerical value according to standard mathematical and/or statistical regulations. For example, a ring's annular segment of "180 degrees" encompasses the angle values of 179 and 181 degrees, more in particular 179.5; 179.6; 179.7; 179.8; 179.9; 180.0; 180.1; 180.2; 180.3; and 180.4 degrees. When this is used in combination with the term "about", said ring's annular segment also encompasses angles that differ from said exact angle by 0.1, 0.2, 0.3, 0.4, 0.5, 0.6, 0.7, 0.7, 0.9, or 1.0 degree. The skilled person will appreciate that when the dimensions of the sustained release intraocular drug delivery device are expressed in length units, the same principle applies; for example a length of "14.0 mm" encompasses length values of 13 to 15 mm, more in particular of 13.5; 13.6; 13.7; 13.8; 13.9; 14.0; 14.1; 14.2; 14.3; and 14.4 mm. Thus, the term "about 14.0 mm" encompasses lengths that differ from said exact length by 0.1, 0.2, 0.3, 0.4, 0.5, 0.6, 0.7, 0.7, 0.9, or 1.0 mm.

Similarly, when a period of time is indicated using a numerical value such as "about 30 minutes", said value encompasses the exact time indicated as well as periods that deviate therefrom by e.g. less than one minute, half a minute, or 1, 2, 3, 4 or 5 minutes. Analogously, the term "about 30 seconds" encompasses about 20, 25 seconds to about 35, 40 seconds, as well as any time in between.

As used throughout the present disclosure, the terms "concentration" and "content" are used interchangeably and refer to the weight concentration or mass fraction of a constituent, i.e. the mass of a constituent divided by the total mass of all constituents, and is expressed in % by weight or % w/w.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Reference throughout this specification to "one embodiment" or "an embodiment" means that a particular feature, structure or characteristic described in connection with the embodiment is included in at least one embodiment of the present application. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments.

By means of further guidance, definitions for the terms used in the description are included to better appreciate the teachings of the present application.

The sustained release intraocular drug delivery device of the invention is defined in the claims.

Preferably, the device comprises or is formed of a compliant annular segment configured for the sulcus of the eye. More specifically, said device has a cross sectional diameter ranging from 0.10 to 0.80 mm. The annular segment can be ranging from 90 to 360° of the ring. Preferably, said annular segment ranges from 180 to 360°, more preferably from 300 to 360° of the ring. In this preferred configuration the eyelet is attached to the elongated body of the device in two attaching points, wherein the two attaching points (4, 5) seal the two ends of the elongate body of the device, so that the device forms a 360° ring, even if the elongated body fails short to form a 360° ring. The annular segment may have an external or outer diameter ranging from 5.0 to 15.0 mm, preferably from 7.5 to 15.0 mm, more preferably from 9.0 to 14.0 mm or from 10.0 to 12.0 mm, such as about 11.0 mm. With the "external diameter" or "outer diameter" of an annulus or an annular segment is meant herein the diameter of the outer circle of the annulus or the annulus segment.

A "sustained release" dosage form is designed to control the drug release for a specific period of time.

As used herein, the term "therapeutic agent" or "compound" is used interchangeably with "drug", and refers to a chemical substance used in the treatment, cure, prevention, or diagnosis of a disease. An example of such therapeutic agents, although the invention is not limited to the following, is for instance Antiangiogenic compounds, such as bevacizumab, ranibizumab, aflibercept, vorolanib, sunitinib (maleate) and axitinib, Beta blockers, such as timolol (maleate), Prostaglandin analogs, such as latanoprost, bimatoprost, travoprost, latanoprostene bunod, tafluprost and omidenepag isopropyl, Carbonic anhydrase inhibitors, such as dorzolamide (hydrochloride) and brinzolamide, Alpha 2 adrenergic agonists, such as brimonidine (tartrate), Rho kinase inhibitors, such as netarsurdil mesylate and ripasudil, Corticosteroids, such as triamcinolone, dexamethasone, difluprednate loteprednol etabonate, fluorometholone hydrocortisone and fluocinolone acetonide, NSAID, such as nepafenac, bromfenac, diclofenac, ketorolac, indomethacin and pranoprofen, Antibiotics, such as moxifloxacin, tobramicine, levofloxacin, gatifloxacin, ofloxacin, besifloxacin, erythromycin, gentamycin, chloramphenicol, azythromycin, polymyxin B sulfate and Bacitracin, or cyclosporin. A general listing may thus comprise the following:, anti-glaucoma agents (i.e. Prostaglandin analogues, Carbonic anhydrase inhibitors, Alpha 2 adrenergic receptors, Beta blockers, Rho kinase inhibitors), anti-inflammatory agents, anti-angiogenesis compounds, and/or immune system modifying agents.

As used herein, an "intraocular drug delivery device" refers to an apparatus that is structured, sized, or otherwise configured to be placed in an eye; and once placed, locally release a drug of choice. Intraocular drug delivery devices of the present application may be biocompatible with physiological conditions of an eye and do not cause adverse side effects in normal eyes and/or in pseudophakic eyes. Intraocular drug delivery devices according to the present application may be placed in an eye without disrupting vision of the eye.

As used herein, a "pseudophakic eye" refers to an eye in which an intraocular lens has been implanted. The intraocular lens may replace a crystalline lens that is damaged, due to for example a perforating wound or ulcer; or does not function properly, for example in the case of cataracts or myopia. The intraocular lens may be also implanted in patients whose crystalline lens is absent due to congenital anomaly.

The "matrix core" of the sustained release intraocular drug delivery device of the present application is located in the innermost part of the present device. It comprises one or more materials, which are insoluble and inert in ocular fluids (i.e. biocompatible) while at the same time they are not absorbed or degraded by the eye tissues. The use of rapidly dissolving materials or materials highly soluble in eye fluids is to be avoided since dissolution of the wall would affect the constancy of the drug release, as well as the capability of the device to remain in place for a prolonged period of time. Preferred materials used for the matrix core of the sustained release intraocular drug delivery device of the present application include, for example polymers.

The matrix core of the sustained release intraocular drug delivery device of the present invention is characterized in that the therapeutic agent is dispersed or distributed therethrough, i.e. the at least one therapeutic agent is mixed into the matrix core, preferably the polymeric matrix core. Dispersion of the therapeutic agent into the matrix core can be achieved by e.g. cross-linking a blend of a crosslinkable polymer (e.g. PDMS) and the at least one therapeutic agent, or by extrusion of a thermoplastic polymer (e.g. EVA, PE, PMMA) and the at least one therapeutic agent.

The matrix core of the sustained release intraocular drug delivery device of the present application is made of a polymer.

As used herein, the term "polymer" refers to a molecule whose structure is composed of multiple repeating units. A "biocompatible polymer" is thus a polymer that is tolerated by living organisms. It may be of natural or synthetic origin.

The materials used in the intraocular drug delivery device according to the invention are selected specifically to ensure that said device forms a compliant annular segment. That is, the annular segment can be straightened by applying force, but will return to its original annular shape when force is no longer applied. This is important, since this enables the simple insertion of the device into the eye, i.e. by temporarily linearising it, after which is returns to its original shape and follows the anatomy of the sulcus of the eye after being inserted. The particular dimensions of the sustained release intraocular drug delivery device of the present application allow for its ease of insertion into the eye and removal after use.

Various polymers can be used to form the polymeric matrix core having the at least one therapeutic agent distributed therethrough. Preferably, the polymer is chemically compatible with the therapeutic agent and permeable to the therapeutic agent.

In an embodiment, the polymeric matrix core comprises a polymer selected from the group consisting of ethylene-co-vinylacetate (EVA), poly(dimethylsiloxane) (PDMS), polypropylene (PP), polyethylene (PE), (plasticized) polyethylene terephthalate (PET), poly(methyl methacrylate) (PMMA), polyvinyl acetate, cross-linked polyvinyl alcohol, cross-linked polyvinyl butyrate, ethylene ethylacrylate copolymer, polyethyl hexylacrylate, polyvinyl alcohol, polyvinyl esters, polyvinylbutyrate, polyamides, polybutylmethacrylate, plasticized nylon, plasticized soft nylon, polyisoprene, cross-linked polyvinylpyrrolidone, poly(1,4-isopropylidene diphenylene carbonate), silicone rubbers, ethylene-propylene rubber, silicone-carbonate copolymers, polycarbonate, polyurethane (preferably thermoplastic), thermoplastic elastomers (TPE) (such as e.g. SEBS (styrene-ethylene-butylene-styrene), SBS (styrene-butadiene-styrene), MBM (methylmethacrylate-butadiene-methyl methacrylate), polyolefins, or combinations thereof.

In a particular embodiment, the polymeric matrix core comprises a polymer selected from the group consisting of ethylene-co-vinylacetate (EVA), poly(dimethylsiloxane) (PDMS), polypropylene, polyethylene), poly(methyl methacrylate) (PMMA), polyurethane (preferably thermoplastic) and crosslinked polyvinyl alcohol, or combinations thereof, wherein at least one therapeutic agent is dispersed throughout said polymer (i.e. wherein at least one therapeutic agent is mixed with said polymer).

The polymeric matrix core of the sustained release intraocular drug delivery device of the present invention also comprises at least one (but potentially two or more) therapeutic agent. The polymeric matrix core of the sustained release intraocular drug delivery device of the present invention may comprise from 0.1 to 50% by weight of the at least one therapeutic agent based on the total weight of polymeric matrix material. Preferably, the polymeric matrix core comprises from 1.0 to 50% or 5.0 to 50% by weight of said at least one therapeutic agent based on the total weight of polymeric matrix material.

The matrix core or polymeric matrix core or the sustained release intraocular drug delivery device of the present invention may also comprise a supporting structure to increase the flexibility of said device. Preferably, said supporting structure is a filament. More preferably, said structure is a metallic filament.

In an embodiment, the matrix core or polymeric matrix core of the sustained release intraocular drug delivery device of the present application comprises a metallic filament.

The sustained release intraocular drug delivery device of the present application also comprises a "polymeric coating" completely surrounding the matrix material, which further regulates the release of the therapeutic agent contained in the polymeric matrix material. Said polymeric coating comprises a polymer, which is insoluble and inert in ocular fluids (i.e. biocompatible), while at the same time it is not absorbed or degraded by the eye tissues. Preferably, the polymer of said polymeric coating is permeable to the therapeutic agent.

In an embodiment, the polymeric coating comprises one or more polymers selected from the group consisting of ethylene-co-vinylacetate (EVA), poly(dimethylsiloxane) (PDMS), polypropylene (PP), polyethylene (PE), (plasticized) polyethylene terephthalate, poly(methyl methacrylate) (PMMA), crosslinked polyvinyl alcohol, polyolefins; regenerated, insoluble, nonerodible cellulose, acylated cellulose, esterified celluloses, cellulose acetate propionate, cellulose acetate butyrate, cellulose acetate phthalate, cellulose acetate diethyl-aminoacetate; polyurethanes (preferably thermoplastic), polycarbonates, and microporous polymers formed by co-precipitation of a polycation and a polyanion modified insoluble collagen, or combinations thereof.

In a particular embodiment, the coating comprises a polymer selected from the group consisting of ethylene-co-vinylacetate (EVA), poly(dimethylsiloxane) (PDMS), polypropylene, polyethylene, poly(methyl methacrylate) (PMMA), polyurethanes (preferably thermoplastic) and crosslinked polyvinyl alcohol, or combinations thereof.

In a particular embodiment, the sustained release intraocular drug delivery device comprises:
a) a matrix core comprising a polymer selected from the group consisting of ethylene-co-vinylacetate (EVA), poly(dimethylsiloxane) (PDMS), polypropylene, polyethylene, poly(methyl methacrylate) (PMMA), polyurethanes (preferably thermoplastic) and crosslinked polyvinyl alcohol, or combinations thereof, wherein at least one therapeutic agent, preferably timolol, is mixed with said polymer, and
b) a coating comprising a polymer selected from the group consisting of ethylene-co-vinylacetate (EVA), poly(dimethylsiloxane) (PDMS), polypropylene, poly(methyl methacrylate) (PMMA), polyurethanes (preferably thermoplastic) and crosslinked polyvinyl alcohol, or combinations thereof.

In particular embodiments, the sustained release intraocular drug delivery device comprises:
(a) a matrix core comprising polyethylene (PE), wherein at least one therapeutic agent, preferably timolol, is mixed with said PE, and
(b) a coating comprising polyethylene (PE).

In particular embodiments, the sustained release intraocular drug delivery device comprises:
(a) a matrix core comprising polyethylene (PE), wherein at least one therapeutic agent, preferably timolol, is mixed with said PE, and
(b) a coating comprising poly(methyl methacrylate) (PMMA).

In particular embodiments, the sustained release intraocular drug delivery device comprises:
(a) a matrix core comprising poly(methyl methacrylate) (PMMA), wherein at least one therapeutic agent, preferably timolol, is mixed with said PMMA, and
(b) a coating comprising poly(methyl methacrylate) (PMMA).

In particular embodiments, the sustained release intraocular drug delivery device comprises:
(a) a matrix core comprising poly(methyl methacrylate) (PMMA), wherein at least one therapeutic agent, preferably timolol, is mixed with said PMMA, and,
(b) a coating comprising polyethylene (PE).

In particular embodiments, the sustained release intraocular drug delivery device comprises:
(a) a matrix core comprising poly(methyl methacrylate) (PMMA), wherein at least one therapeutic agent, preferably timolol, is mixed with said PMMA, and
(b) a coating comprising ethylene-co-vinylacetate (EVA).

In particular embodiments, the sustained release intraocular drug delivery device comprises:
(a) a matrix core comprising polyethylene (PE), wherein at least one therapeutic agent, preferably timolol, is mixed with said PE, and
(b) a coating comprising ethylene-co-vinylacetate (EVA).

In particular embodiments, the sustained release intraocular drug delivery device comprises:
(a) a matrix core comprising poly(methyl methacrylate) (PMMA), wherein at least one therapeutic agent, preferably timolol, is mixed into said PMMA, and
(b) a coating comprising polypropylene (PP).

In particular embodiments, the sustained release intraocular drug delivery device comprises:
a) a matrix core comprising ethylene-co-vinylacetate (EVA), wherein at least one therapeutic agent, preferably timolol, is mixed with said EVA, and
(b) a coating comprising polyethylene (PE).

In particular embodiments, the sustained release intraocular drug delivery device comprises:
a) a matrix core comprising ethylene-co-vinylacetate (EVA), wherein at least one therapeutic agent, preferably timolol, is mixed with said EVA, and
(b) a coating comprising poly(methyl methacrylate) (PMMA).

In particular embodiments, the sustained release intraocular drug delivery device comprises:
a) a matrix core comprising ethylene-co-vinylacetate (EVA), wherein at least one therapeutic agent, preferably timolol, is mixed with said EVA, and
b) a coating comprising ethylene-co-vinylacetate (EVA).

In particular embodiments, the sustained release intraocular drug delivery device comprises:
a) a matrix core comprising ethylene-co-vinylacetate (EVA), wherein at least one therapeutic agent, preferably timolol, is mixed with said EVA, and
b) a coating comprising a polyurethane (PU).

In particular embodiments, the sustained release intraocular drug delivery device comprises:
a) a matrix core comprising a polyurethane (PU), wherein at least one therapeutic agent, preferably timolol, is mixed with said PU, and
b) a coating comprising ethylene-co-vinylacetate (EVA).

In particular embodiments, the sustained release intraocular drug delivery device comprises:
a) a matrix core comprising a polyurethane (PU), wherein at least one therapeutic agent, preferably timolol, is mixed with said PU, and
b) a coating comprising a polyurethane (PU).

Further details of the elongate body of the sustained release intraocular drug delivery device could be found in EP3068372 B1. In other words, the technical details regarding dimension, composition, material and manufacturing process for the elongate body of the sustained release intraocular drug delivery device in this document is in line with the disclosure of EP3068372 B1.

Hereunder in this document, it will focus on the design of eyelet comprised in the sustained release intraocular drug delivery device.

The present invention concerns a sustained release intraocular drug delivery device, comprising: an elongate body, two (sealed) ends, an eyelet fixedly connected with at least one end, wherein said body further comprises a polymeric matrix core into which at least one therapeutic agent is mixed, and a polymeric coating completely surrounding said polymeric matrix material, wherein said eyelet is configured to assist the withdrawal of said device.

The eyelet, and preferably the ends, are free of therapeutic agent. The two (sealed) ends and the eyelet are composed from at least one polymer that are compatible with the polymer included in the sustained release intraocular drug delivery device. The polymer could be the core polymer, the coating polymer or another polymer. There is no chemical reaction between the material of eyelet, two (sealed) ends and the body of the sustained release intraocular drug delivery device.

In the invention, the eyelet preferably comprises at least a linking portion and a gripping portion, said gripping portion preferably comprising an opening therethrough, the linking portion connecting the eyelet to at least one, preferably both, of the (sealed) ends, and the gripping means extending therefrom, away from the body of the device and adapted to be manipulated by a user for removal (and possibly for adjustment of position in the eye).

In a preferred embodiment, the eyelet comprises a narrowed section, said narrowed section either being comprised in the linking portion or being positioned between the gripping portion and the linking portion, said narrowed section allowing the eyelet to be folded against the ring body for linearization. In a further preferred embodiment, said narrowed section has a minimal cross-sectional dimension lower than that of the body, and preferably also lower than that of the gripping portion.

In specifically preferred embodiments, the minimal cross-sectional dimension lies along the direction essentially perpendicular to the eyelet opening (as in the direction of a line extending through the opening). However, the minimal width of the narrowed section (in the plane wherein the opening lies) is preferably also at most equal to (or lower than) the cross-section dimension of the elongate body. Most preferably said minimal width and the minimal cross-sectional dimension of the narrowed section are present at overlapping parts of said narrowed section. The reduction of said dimension further simplifies the folding of the eyelet.

Indeed, preferably, the device is folded before application to a patient, so as to avoid large incisions. The folded device is thus almost linear. Such a folding is achieved upon a gentle longitudinal compression.

In other words, preferably, in the context of the present invention, the wordings "compression", "linearization" and "folding" do refer to the same action. This allows a placement in a compact (very narrow) configuration through a simple surgery.

In a preferred embodiment, the gripping portion is at the position of the opening at least about as wide, and preferably wider, in its entirety in comparison to where said gripping portion connects to the linking portion, to improve linearization.

In a particular embodiment, the elongate body has a maximal cross-sectional diameter of 0.8 mm, preferably at most 0.7 mm, 0.6 mm or even 0.5 mm. In the most preferred embodiment, the elongate body has a cross sectional diameter between 0.2 mm and 0.4 mm, for instance between about 0.30 mm and 0.35 mm, or between about 0.25 and 0.30 mm. This much thinner configuration particularly differs from the prior art, further necessitating the need for an eyelet to withdraw the device, as it is more fragile and could be damaged in handling it directly (potentially leaving exposed polymer matrix material comprising an agent in the eye), while the thinness also makes handling more difficult in general.

In a particular embodiment, the eyelet extends from the elongate body over at least 1.0 mm, preferably at least 1.25 mm and more preferably at least 1.5 mm, even at least 1.7 mm, 1.8 mm, 1.9 mm 2.0 mm or (about) 2.1 mm, ensuring both an appropriate length for easy pick-up during the withdrawal of the device without potentially damaging the elongate body. Furthermore, it is important that the eyelet is visible under mydriasis while not disrupting vision of the eye, which is accomplished by the minimal distance set above. Note that this mainly (over 80%) relates to the length of the so-called gripping portion, as can be seen in the Figures.

Conversely, the applicant noted that preferably, a maximal length of the eyelet extending from the elongate body should be set at about 4.5 mm, preferably about 4.0 mm and more preferably about 3.5 mm, preferably about 3.0 mm.

As such, most preferably, the eyelet preferably extends over a distance between 1.5 mm and 3.5 mm from the elongate body, giving the perfect balance between easy detection under mydriasis, no negative effects on eyesight of the patient, and easy pick-up.

In a particular embodiment, the eyelet comprises a linking portion and a gripping portion, whereby the eyelet is attached to the elongate body via the linking portion, which functions as a base from which the eyelet further extends. The linking portion preferably is oriented as a continuation of the elongate body, with the gripping portion of the eyelet comprising the opening extending perpendicularly from said linking portion.

In a further preferred embodiment, the eyelet further comprises a junction between the gripping portion and the linking portion, said junction comprising the narrowed section that is thinner in width than the gripping portion. Especially, the gripping portion forms an elongate extension with a length much higher than its width. All of the measures taken in these embodiments allows high flexibility of the eyelet and linearization (and/or folding) with the elongate body for insertion and removal of the implant. The width of the junction is most preferably between about 40% to about 75% of the width of the gripping portion, for instance between 0.2 mm to 1.0 mm compared to the width of the gripping portion between 0.5 mm and 1.4 mm. Preferably, the ratio is between about 50% and 60%, most preferably about 55% (for instance a range of 0.3 mm to 0.55 mm versus a range of 0.6 mm to 0.9 mm, or about 0.41 to about 0.74 mm).

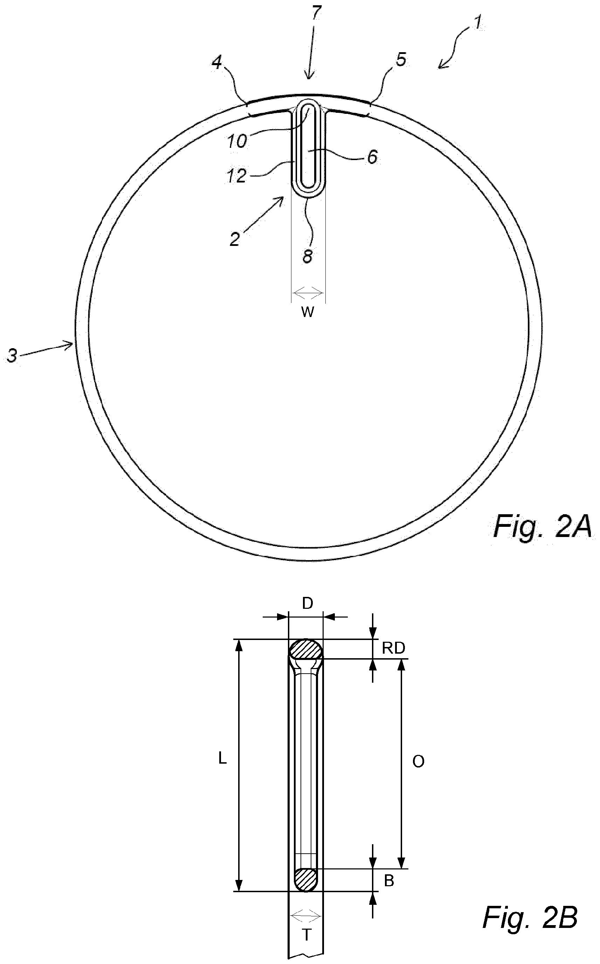

In an alternatively preferred embodiment (Figure 2A), the gripping portion comprises an opening which extends partly into the linking portion of the eyelet, resulting in a section of the linking portion with a reduced cross-sectional dimension in view of surrounding sections of the linking portion (and typically with a lower cross-sectional dimension than the body of the device), this to optimize linearization. Preferably, the ratio of the section with reduced cross-sectional dimension of the linking portion compared to the cross-sectional dimension of its neighbouring sections is between about 45% and 70%, preferably about 50% to 65%, more preferably about 55% to 60%. The opening extends into the linking portion, thereby defining the narrowed section, whereby said narrowed section has a minimal width in the dimension along which the gripping portion extends from the elongate body, which minimal width of the narrowed section is lower than the minimal width in said dimension of the surrounding sections of the linking portion. Note that this particular embodiment allows the body to fold against the eyelet itself.

It should be noted that a combination of any two or more of the above embodiments as well as those following, is considered to form part of the invention, said combinations providing particularly preferable embodiments of the invention.

In a preferred embodiment, the two (sealed) ends and the eyelet are composed of a single polymer. In another preferred embodiment, the two (sealed) ends and the eyelet are composed of a combination of two polymers or more. It needs to be noted that the composition of polymer or polymers used in one (sealed) end, the other (sealed) end and those used in the eyelet are completely independently from each other.

In some embodiments, the two (sealed) ends are comprised in the eyelet, in the sense that they are directly connected thereto. In some embodiments, one (sealed) end is comprised in the eyelet, and the other one is not. In some embodiments, one (sealed) end, the other (sealed) end and the eyelet are separated from each other.

The manufacturing process of the eyelet can be achieved by several technologies known by the skilled person. The maximal of the melting temperature of the polymer comprised in the eyelet is preferably 200°C.

To assist the withdrawal of the sustained release intraocular drug delivery device, the eyelet comprises an opening allowing to introduce a classical instrument in order to pick-up the implant and remove it out of the eye. The opening secures the withdrawal of said drug delivery device. In order to be visible by the physician, but not affecting the injectability, the diameter of the opening is ranging from 0.1 to 1 mm, and preferably between 0.2 to 0.8 mm, more preferably between 0.25 mm and 0.6 mm, and even more preferably between 0.3 mm and 0.45 mm.

To define such an opening, the material is distributed as two arms, which have a minimal cross-sectional dimension of 0.15 mm, preferably 0.2 mm considering the manufacturing process. Preferably, the arms have a maximal cross-sectional dimension/thickness of 0.8 mm, more preferably at most 0.6 mm or even at most 0.4 mm. In general, thinner arms are preferred as long as strength is maintained to allow for the handling of the eyelet. The disclosed width range ensures sufficient stiffness to maintain the opening, and enough flexibility to allow handling, while also ensuring minimal hinder to the patient.

Considering the opening and the arms defining it, the transversal dimension of the gripping portion in the plane defined by the opening ranges between 0.5 to 1.4 mm, preferably between 0.6 to 0.9 mm, and more preferably is between 0.7 and 0.8 mm. Note that the linking portion, as a continuation of the body of the device itself, can have a greater transversal dimension in said plane.

To better assist the withdrawal of the drug delivery device but also avoid any possible injury to the eye and damage to the eyelet itself, the eyelet shall also follow certain rules regarding the mechanical resistance. In the current application, the minimal resistance is 0.1 Newton, 0.5 N or even values closer to 1 N.

The injection/implantation of the drug delivery device is preferably achieved by an injector, which requires the drug delivery device to be "linearized". To avoid affecting this feature, in a preferred embodiment, the junction where the linking portion and the gripping portion are connected, shall comprise a narrowed section that has a width (in the plane of the eyelet) lower than the diameter of cross section of the elongate body and/or than the gripping portion of the eyelet. Moreover, the eyelet thickness (dimension perpendicular to the eyelet) shall preferably be smaller than the diameter of cross section of the elongate body.

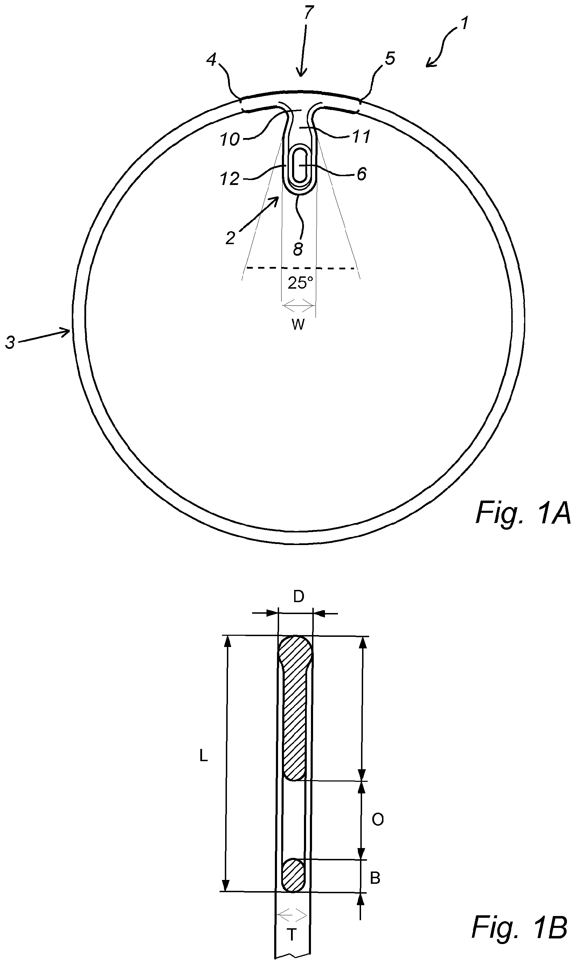

Fig. 1A shows a preferred embodiment of the sustained release intraocular drug delivery device comprising a first version of an eyelet, with Fig. 1B showing dimensional details.

The elongate body (3) is configured to form a non-closed ring, which is further processed to form a closed loop, preferably a torus. The eyelet (2) is configured to complete the ring, connected with the body (3) of the sustained release intraocular drug delivery device (1) at two attaching points (4, 5), wherein the two attaching points also seal the two ends of the elongate body (3) respectively. The eyelet further comprises a gripping portion (8) with an opening (6) and a linking portion (7), which links said gripping portion (comprising the opening) to said attaching points, wherein the tip of the gripping portion (8) is pointing towards the centre of the ring. The eyelet comprises a junction (10) linking said gripping portion (8) and said linking portion (7), wherein the linking portion (7) is configured with the same dimension in cross section as the elongate body (3). The junction (10) comprises a narrowed section (11) close to the linking portion (7), allowing the eyelet to be folded against the ring body for linearization.

Fig. 2A shows a second preferred embodiment of the sustained release intraocular drug delivery device comprising a second version of an eyelet.

The elongate body (3) is configured to form a non-closed ring, which is further processed to form a closed loop, preferably a torus. The eyelet (2) is configured to complete the ring, connected with the body (3) of the sustained release intraocular drug delivery device (1) at two attaching points (4, 5), wherein the two attaching points also seal the two ends of the elongate body (3) respectively. The eyelet further comprises a gripping portion (8), comprising an opening (6), and a linking portion (7), which links said gripping portion to the attaching points, wherein the end tip of the gripping portion (8) is pointing towards the centre of the ring. The eyelet comprises a junction (10) linking the gripping portion (8) to the linking portion (7). Note that the linking portion (7) is generally configured with the same dimension in cross section as the elongate body (3), excepting the narrowed section where the opening extends into. Importantly, it should be noted that the junction (10) is narrowed where the linking portion (7) connects to the gripping portion (8), specifically between the two points of connection. At this position, the opening (6) of the gripping portion (8) extends into the linking portion (7). This is shown in Fig. 2A for instance, as well as in the side view of Fig. 2B. In particular, the reduction in the cross-sectional dimension of the linking portion in the plane defined by the opening (6) is at least 25%, preferably even at least 33% or 40%, as this allows for improved linearization. For instance, in Figures 2A-2B, this is shown in the cross-sectional dimension of the ring itself being about 0.33 mm, while at the junction, the cross-sectional dimension where the opening (6) extends into the linking portion, is about 0.19 mm.

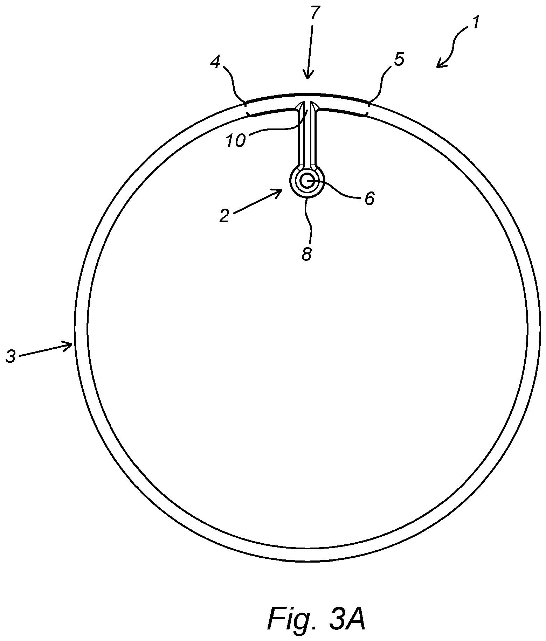

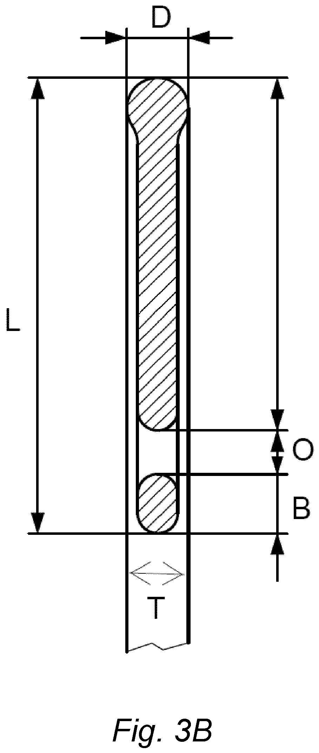

Fig. 3A and 3B show a third preferred embodiment of the sustained release intraocular drug delivery device comprising a third version of an eyelet.

The elongate body (3) is configured to form a non-closed ring, which is further processed to form a closed loop, preferably a torus. The eyelet (2) is configured to complete the ring, connected with the body (3) of the sustained release intraocular drug delivery device (1) at two attaching points (4, 5), wherein the two attaching points also seal the two ends of the elongate body (3) respectively. The eyelet further comprises a gripping portion (8), which comprises an opening (6) which may or may not be a round circle, and a linking portion (7), which links the gripping portion to the ring at the attaching points, wherein the tip of the gripping portion (8) is pointing towards the centre of the ring. The linking portion (7) is connected to the gripping portion (8) via a junction (10). Note that the linking portion (7) is preferably configured with the same dimension in cross section as the elongate body (3) and the junction (10) is narrowed on all parts between the linking portion (7) and the gripping portion (8), allowing the eyelet to be folded against the ring body (3) for linearization. Further to that point, the transition from the junction to the linking portion may itself be rounded, as can be seen in the figures.

### EXAMPLE

A preferred embodiment of the present invention is presented below.

### EXAMPLE 1

Fig. 1A and Fig. 1B show a first preferred embodiment of the sustained release intraocular drug delivery device comprising a first version of an eyelet.

The elongate body (3) is configured to form a non-closed nearly 360° ring shape (e.g. 350°). The eyelet (2) is configured to fulfil the ring, connected with the body (3) of the sustained release intraocular drug delivery device (1) at two attaching points (4, 5), wherein the two attaching points also seal the two ends of the elongate body (3) respectively. The eyelet further comprises a gripping portion (8) and a linking portion (7), wherein the end tip of the gripping portion (8) is pointing towards the centre of the ring. The linking portion (7) is essentially a continuation of the ring itself, connecting the two ends of the body (3) to each other. The linking portion is linked to the gripping portion via a junction (10) comprising a narrowed section (11).

The eyelet (including the two attaching points) is free of therapeutic agent and made of polymer compatible with the polymer included in the drug delivery device, comprising the core polymer, the coating polymer or another polymer.

Fig. 1B shows the dimensional details of the sustained release intraocular drug delivery device (1). The elongate cylinder body has a diameter D of 0.33 mm in cross section, and forms a ring with an outer diameter of about 11 mm. The eyelet (2) has in total a length L of about 2.50 mm, 0.74 mm in width W (see Fig. 1A), and 0.22 mm in thickness T, which is thinner than the cross section of the linking portion (7), which further favours linearization into the injector device (Fig. 1B). The eyelet is plane-symmetrical with respect to a plane perpendicular to the body through the longitudinal axis of the eyelet, plane-symmetrical to a plane parallel to the body thereof and through the longitudinal axis of the eyelet, and fully rounded in cross section.

The opening (6) is about 0.77 mm (O) in the length and about 0.30 mm in width (transversal). The bottom of opening (6) is about 0.33 mm (B) from the end tip (8). The two arms (12) of the opening (6) along the length are configured to resemble a cylinder with a cross section of about 0.22 mm. However, it should be understood that variations on the shape can exist, such as oval, square, etc. The junction (10) is gradually decreasing its width from the top of the gripping portion (6) to the linking portion (7), wherein the narrowest part could reach 0.41 mm at the narrowed section (11). The junction (10) is rounded at the point where it meets the linking portion (7), preferably in a Radius of 0.20mm circle. Note that the radius of said circle can however vary between 0.10mm and 0.30, preferably between 0.15 and 0.25 mm.

In a most preferred embodiment, the junction (10) diverges from said narrowed section (11) towards the gripping portion (8) at an angle between 20° and 30°, preferably about 25°, as can be seen in Fig. 1A.

### EXAMPLE 2

Fig. 2A and Fig. 2B show a second preferred embodiment of the sustained release intraocular drug delivery device comprising a second version of an eyelet.

The elongate body (3) is configured to form a non-closed nearly 360° ring shape (e.g. 350°). The eyelet (2) is configured to fulfil the ring, connected with the body (3) of the sustained release intraocular drug delivery device (1) at two attaching points (4, 5), wherein the two attaching points also seal the two ends of the elongate body (3) respectively. The eyelet further comprises a gripping portion (8), comprising an opening (6), and a linking portion (7), wherein the end tip of the gripping portion is pointing towards the centre of the ring. The junction (10) links the gripping portion to the linking portion and is narrowed wherein the linking portion connects to the gripping portion and wherein the linking portion (7) is configured with the same dimension in cross section as the elongate body (3). The opening (6) extends into the linking portion at the junction, as can be seen in Figures 2A-2B. The cross-sectional dimension RD where the opening (6) extends into the linking portion, is about 0.19 mm, as opposed to the diameter D, which is about 0.33 mm, of the body surrounding said reduced section.

The eyelet (including the two attaching points) is free of therapeutic agent and made of polymer compatible with the polymer included in the drug delivery device, comprising the core polymer, the coating polymer or another polymer.

Fig. 2B shows the dimensional details of a detailed embodiment for the sustained release intraocular drug delivery device (1). The elongate cylinder (it should be understood that variations on the shape can exist, such as oval, square, etc.) body has a diameter D of about 0.33 mm in cross section. The eyelet (2) is in total about 2.50 mm in length L, about 0.74 mm in its transversal dimension W (perpendicular to the direction in which it extends from the body, and lying in the plane defined by the opening), and has a thickness T of about 0.22 mm. This means it is thinner than the cross section of the linking portion (7) (Fig. 2B), which further favours linearization into the injector device. The eyelet is plane-symmetrical with respect to a plane perpendicular to the body through the longitudinal axis of the eyelet, plane-symmetrical to a plane parallel to the body thereof and through the longitudinal axis of the eyelet, and substantially fully rounded in cross section.

The opening O (6) is 2.09 mm in the lengthwise dimension and 0.30 mm in the transversal dimension. The two arms of the opening (6) along the length have a cross-sectional diameter B of about 0.22 mm. The junction (10) is rounded at the point where it meets the linking portion (7) in a circle with a radius of 0.10 mm, although this could vary between R0.05 and R0.30mm, preferably between R0.075 and R0.15mm.

### EXAMPLE 3

Fig. 3A and 3B show a third preferred embodiment of the sustained release intraocular drug delivery device comprising a third version of an eyelet.

The elongate body (3) is configured to form a non-closed nearly 360° ring shape (e.g. 350°). The eyelet (2) is configured to fulfil the ring, connected with the body (3) of the sustained release intraocular drug delivery device (1) at two attaching points (4, 5), wherein the two attaching points also seal the two ends of the elongate body (3) respectively. The eyelet further comprises a gripping portion (8) and a linking portion (7), which the end tip (8) of the gripping portion is pointing towards the centre of the ring. The linking portion is connected to the gripping portion via a junction (10) having a narrow width compared to the gripping portion that allows the eyelet to be folded against the ring body for linearization.

The eyelet (including the two attaching points) is free of therapeutic agent and made of polymer compatible with the polymer included in the drug delivery device, comprising the core polymer, the coating polymer or another polymer.

The eyelet is plane-symmetrical with respect to a plane perpendicular to the body through the longitudinal axis of the eyelet, plane-symmetrical to a plane parallel to the body thereof and through the longitudinal axis of the eyelet, and substantially fully rounded in cross section. The gripping portion (8) around the opening (6) is configured as a ring shape. The junction (10) is configured as a cylinder (it should be understood that variations on the shape can exist, such as oval, square, etc.) portion linking the gripping portion and the linking portion. The junction (10) is thinner than the opening and the gripping portion, in favour of linearization into the injector device. Preferably, the junction (10) is rounded at the point where it meets the linking portion (7), more preferably rounded in a circle with a radius that varies between R0.05 and R0.30mm, preferably between R0.075 and R0.15mm.

Fig. 3B shows the dimensional details of the eyelet of the device (1) of Figure 3A. In this embodiment, though these dimensions are not meant to be limiting but only serve as further examples, the length L of the eyelet is similar to that of examples 1 and 2, about 2.5 cm, with an opening O of about 0.3-0.4 cm diameter. The gripping portion (8) around the opening (6) has a cross-sectional diameter T of about 0.22 mm, and the junction (10) has a width D (diameter) of about 0.33 cm. The elongate body is in the form of a circle with a diameter of about 10-11 cm, and the body itself is tubular with a diameter of about 0.33 cm.

### EXAMPLE 4

In example 4, the manufacturing of the sustained release intraocular drug delivery device in preferred embodiments of example 1-3 and/or beyond is demonstrated. The steps comprise:
- providing an elongate body comprising a polymeric matrix core and a polymeric coating completely surrounding said polymeric matrix, wherein at least one therapeutic agent is mixed into the polymeric matrix core and the polymeric coating regulates the therapeutic agent release rates, wherein the two ends of the elongate body are not sealed;
- positioning the elongate body into a mold which forms said elongate body into a 350° ring, wherein said elongate body is provided with the predetermined length to exactly form the such a non-closed ring ;
- adding a polymer into the mold to form the eyelet according to example 1, 2 or 3 and/or any other embodiments of the eyelet according to the description, meanwhile the polymer also seals the two ends of said elongate body, links the two ends and completes the device into a full ring;

### EXAMPLE 5

In example 5, the manufacturing of the sustained release intraocular drug delivery device in preferred embodiments of example 4 and/or beyond is demonstrated. The steps comprises:
- providing an elongate body comprising a polymeric matrix core and a polymeric coating completely surrounding said polymeric matrix, wherein at least one therapeutic agent is mixed into the polymeric matrix core and the polymeric coating regulates the therapeutic agent release rates, wherein one end of the elongate body is sealed and the other one is not;
- positioning the elongate body into a mold which may or may not form said elongate body into any specific shape;
- adding a polymer into the mold to form the eyelet according to example 4 and/or any other embodiments of the eyelet according to the description, meanwhile the polymer also seals the one end of said elongate body that is not sealed in previous step.

## Claims

1. A sustained release intraocular drug delivery device (1), comprising: an elongate body (3) comprising two ends, and an eyelet (2) fixedly connected with at least one end of the elongate body;
wherein said elongate body (3) further comprises a substantially elongate polymeric matrix core into which at least one therapeutic agent is mixed, and a mantle completely surrounding said matrix core, said mantle comprising, and preferably being, a polymeric coating;
wherein said eyelet (2) is adapted to assist in the withdrawal of said device (1) and wherein said eyelet (2) is essentially free of therapeutic agent, and wherein the eyelet comprises a gripping portion (8) and a linking portion (7), said linking portion (7) connecting the gripping portion (8) to at least one end, preferably both ends, of the body.

2. A sustained release intraocular drug delivery device (1) according to claim 1, wherein the gripping portion (8) comprises an opening (6).

3. The sustained release intraocular drug delivery device (1) according to claim 1 or 2, further comprising a narrowed section (11) being comprised in the linking portion (7) or being positioned between the gripping portion (8) and the linking portion (7), the said narrowed section (11) facilitating the folding of the said drug delivery device (1).

4. A sustained release intraocular drug delivery device (1) according to any one of the preceding claims 1 to 3, wherein the ends (4,5) of the elongate body (3) are sealed, and the sealed ends (4,5) are free of therapeutic agent, wherein at least one sealed end (4,5) is sealed by the eyelet (2), preferably the two ends (4,5) are sealed by the eyelet (2).

5. A sustained release intraocular drug delivery device (1) according to any one of the preceding claims 1 to 4, wherein the eyelet (2) comprises one or more of the following polymers: ethylene-vinyl acetate (EVA), poly(dimethylsiloxane) or PDMS, polyolefins, poly-ethylene, polyurethane, poly(methyl methacrylate) or PMMA, and crosslinked polyvinyl alcohol or a combination thereof.

6. A sustained release intraocular drug delivery device (1) according to claim 5, wherein the polymer composing the eyelet (2) is compatible with the polymer included in the elongate body (3), said polymer of the elongate body (3) comprising: ethylene-vinyl acetate (EVA), poly(dimethylsiloxane) or PDMS, polyolefins, poly-ethylene, polyurethane, poly(methyl methacrylate) or PMMA, thermoplastic polyurethane (TPU), and crosslinked polyvinyl alcohol or a combination thereof.

7. A sustained release intraocular drug delivery device (1) according to any one of the preceding claims 1 to 6, wherein the eyelet (2) further comprises a junction (10) connecting the gripping portion (8) and the linking portion (7), wherein said junction (10) comprises the narrowed section (11).

8. A sustained release intraocular drug delivery device (1) according to any one of claims 1 to 7, wherein the gripping portion (8) and the linking portion (7) are directly connected, and wherein the opening (6) in the gripping portion (8) partly extends into a section of the linking portion (7), thereby defining the narrowed section (11), whereby said narrowed section (11) has a minimal width in the dimension along which the gripping portion (8) extends from the elongate body (3), which minimal width of the narrowed section (11) is lower than the minimal width in said dimension of the surrounding sections of the linking portion (7).

9. A sustained release intraocular drug delivery device (1) according to any one of the preceding claims 1 to 8, wherein said device (1) is a 360° ring and the eyelet (2) is attached to said device (1) in two attaching points (4, 5), wherein the two attaching points (4, 5) seal the two ends of the elongate body (3) of said device (1), respectively, preferably, wherein the eyelet (2) protrudes towards the center of the ring.

10. A sustained release intraocular drug delivery device (1) according to any one of the preceding claims 1 to 9, wherein said device (1) is in a folded configuration suitable for injecting into the eye with an injecting device.

11. A sustained release intraocular drug delivery device (1) according to any one of the preceding claims 1 to 10, wherein the eyelet (2) extends from the elongate body (3) over a distance ranging from 1.0 mm to 3.5 mm, preferably from 1.5 mm to 3.0 mm, more preferably between 1.7 mm to 2.7 mm, still more preferably between 2,0 and 2,6 mm.

12. A sustained release intraocular drug delivery device (1) according to any one of the preceding claims 1 to 11, wherein the maximal width dimension of the opening (6) in said eyelet ranges from 0.1mm to 1.0 mm, preferably from 0.2mm to 0.6mm, more preferably from 0.25 mm to 0.4 mm.

13. A sustained release intraocular drug delivery device (1) according to any one of the preceding claims 1 to 12, wherein the opening (6) is defined by two arms which join at their respective ends, wherein the minimal width of the arms is 0.15 mm.

14. A sustained release intraocular drug delivery device (1) according to any one of the preceding claims 1 to 13, wherein the transversal dimension of the gripping portion (8) in the plane defined by the opening (6) ranges from 0.5 mm to 1.4mm, preferably from 0.6 mm to 0.9 mm, most preferably about 0.7 mm to 0.8 mm.

15. A process for preparing a removable sustained release intraocular drug delivery device, preferably according to any one of the preceding claims 1 to 14, comprising:
- providing an elongate body (3) comprising a polymeric matrix core and a polymeric coating completely surrounding said polymeric matrix, wherein at least one therapeutic agent is mixed into the polymeric matrix core, wherein the two ends of the elongate body are not sealed;
- positioning the elongate body into a mold which forms said elongate body into a nearly 360° ring;
- adding at least one polymer to form the eyelet (2), wherein the polymer also seals the two ends (4, 5) of said elongate body (3).

## Patentansprüche

1. Eine Intraokulare Arzneimittelabgabevorrichtung (1) mit kontinuierlicher Freisetzung, umfassend: einen länglichen Körper (3), der zwei Enden umfasst, und eine Öse (2), die fest mit mindestens einem Ende des länglichen Körpers verbunden ist,
wobei der längliche Körper (3) ferner einen im Wesentlichen länglichen Polymermatrixkern, in den mindestens ein therapeutisches Mittel gemischt ist, und einen Mantel, der den Matrixkern vollständig umgibt, umfasst, wobei der Mantel eine Polymerbeschichtung umfasst und vorzugsweise ein solcher ist,
wobei die Öse (2) dafür eingerichtet ist, das Entnehmen der Vorrichtung (1) zu unterstützen, und wobei die Öse (2) im Wesentlichen frei von therapeutischem Mittel ist und wobei die Öse einen Griffabschnitt (8) und einen Kopplungsabschnitt (7) umfasst, wobei der Kopplungsabschnitt (7) den Griffabschnitt (8) mit mindestens einem Ende, vorzugsweise mit beiden Enden, des Körpers verbindet.

2. Eine Intraokulare Arzneimittelabgabevorrichtung (1) mit kontinuierlicher Freisetzung nach Anspruch 1, wobei der Griffabschnitt (8) eine Öffnung (6) umfasst.

3. Die Intraokulare Arzneimittelabgabevorrichtung (1) mit kontinuierlicher Freisetzung nach Anspruch 1 oder 2, ferner einen verengten Teilabschnitt (11) umfassend, der in dem Kopplungsabschnitt (7) enthalten oder zwischen dem Griffabschnitt (8) und dem Kopplungsabschnitt (7) positioniert ist, wobei der verengte Teilabschnitt (11) das Falten der Arzneimittelabgabevorrichtung (1) erleichtert.

4. Eine Intraokulare Arzneimittelabgabevorrichtung (1) mit kontinuierlicher Freisetzung nach einem der vorhergehenden Ansprüche 1 bis 3, wobei die Enden (4, 5) des länglichen Körpers (3) versiegelt sind und die versiegelten Enden (4, 5) frei von therapeutischem Mittel sind, wobei mindestens ein versiegeltes Ende (4, 5) durch die Öse (2) versiegelt ist, vorzugsweise die zwei Enden (4, 5) durch die Öse versiegelt sind.

5. Eine Intraokulare Arzneimittelabgabevorrichtung (1) mit kontinuierlicher Freisetzung nach einem der vorhergehenden Ansprüche 1 bis 4, wobei die Öse (2) eines oder mehrere der folgenden Polymere umfasst: Ethylenvinylacetat (EVA), Poly(dimethylsiloxan) oder PDMS, Polyolefine, Polyethylen, Polyurethan, Poly(methylmethacrylat) oder PMMA und vernetzten Polyvinylalkohol oder eine Kombination davon.

6. Eine Intraokulare Arzneimittelabgabevorrichtung (1) mit kontinuierlicher Freisetzung nach Anspruch 5, wobei das Polymer, das die Öse (2) bildet, mit dem Polymer kompatibel ist, das in dem länglichen Körper (3) enthalten ist, wobei das Polymer des länglichen Körpers (3) Folgendes umfasst: Ethylenvinylacetat (EVA), Poly(dimethylsiloxan) oder PDMS, Polyolefine, Polyethylen, Polyurethan, Poly(methylmethacrylat) oder PMMA, Thermoplastpolyurethan (TPU) und vernetzten Polyvinylalkohol oder eine Kombination davon.

7. Eine Intraokulare Arzneimittelabgabevorrichtung (1) mit kontinuierlicher Freisetzung nach einem der vorhergehenden Ansprüche 1 bis 6, wobei die Öse (2) ferner einen Knotenpunkt (10) umfasst, der den Griffabschnitt (8) und den Kopplungsabschnitt (7) verbindet, wobei der Knotenpunkt (10) den verengten Teilabschnitt (11) umfasst.

8. Eine Intraokulare Arzneimittelabgabevorrichtung (1) mit kontinuierlicher Freisetzung nach einem der Ansprüche 1 bis 7, wobei der Griffabschnitt (8) und der Kopplungsabschnitt (7) direkt verbunden sind und wobei sich die Öffnung (6) in dem Griffabschnitt (8) teilweise in einen Teilabschnitt des Kopplungsabschnitts (7) hinein erstreckt, wodurch der verengte Teilabschnitt (11) definiert ist, wobei der verengte Teilabschnitt (11) in der Abmessung, über die sich der Griffabschnitt (8) von dem länglichen Körper (3) aus erstreckt, eine minimale Breite aufweist, wobei die minimale Breite des verengten Teilabschnitts (11) geringer als die minimale Breite in der Abmessung der umgebenden Teilabschnitte des Kopplungsabschnitts (7) ist.

9. Eine Intraokulare Arzneimittelabgabevorrichtung (1) mit kontinuierlicher Freisetzung nach einem der vorhergehenden Ansprüche 1 bis 8, wobei die Vorrichtung (1) ein 360°-Ring ist und die Öse (2) an zwei Anbringungspunkten (4, 5) an der Vorrichtung (1) angebracht ist, wobei die zwei Anbringungspunkte (4, 5) die zwei Enden des länglichen Körpers (3) der Vorrichtung (1) entsprechend versiegeln, wobei die Öse (2) vorzugsweise hin zur Mitte des Rings hervorsteht.

10. Eine Intraokulare Arzneimittelabgabevorrichtung (1) mit kontinuierlicher Freisetzung nach einem der vorhergehenden Ansprüche 1 bis 9, wobei die Vorrichtung (1) in einer zusammengefalteten Gestaltung zur Injektion in das Auge mit einer Injektionsvorrichtung geeignet ist.

11. Eine Intraokulare Arzneimittelabgabevorrichtung (1) mit kontinuierlicher Freisetzung nach einem der vorhergehenden Ansprüche 1 bis 10, wobei sich die Öse (2) von dem länglichen Körper (3) über eine Distanz im Bereich von 1,0 mm bis 3,5 mm erstreckt, vorzugsweise von 1,5 mm bis 3,0 mm, bevorzugter zwischen 1,7 mm und 2,7 mm, noch bevorzugter zwischen 2,0 und 2,6 mm.

12. Eine Intraokulare Arzneimittelabgabevorrichtung (1) mit kontinuierlicher Freisetzung nach einem der vorhergehenden Ansprüche 1 bis 11, wobei die maximale Breite-Abmessung der Öffnung (6) in der Öse im Bereich von 0,1 mm bis 1,0 mm liegt, vorzugsweise von 0,2 mm bis 0,6 mm, bevorzugter von 0,25 mm bis 0,4 mm.

13. Eine Intraokulare Arzneimittelabgabevorrichtung (1) mit kontinuierlicher Freisetzung nach einem der vorhergehenden Ansprüche 1 bis 12, wobei die Öffnung (6) durch zwei Arme definiert ist, die an ihren jeweiligen Enden zusammenlaufen, wobei die minimale Bereite der Arme 0,15 mm beträgt.

14. Eine Intraokulare Arzneimittelabgabevorrichtung (1) mit kontinuierlicher Freisetzung nach einem der vorhergehenden Ansprüche 1 bis 13, wobei die Querabmessung des Greifabschnitts (8) in der Ebene, die durch die Öffnung (6) definiert ist, im Bereich von 0,5 mm bis 1,4 mm liegt, vorzugsweise von 0,6 mm bis 0,9 mm, am meisten bevorzugt etwa 0,7 mm bis 0,8 mm.

15. Ein Verfahren zur Herstellung einer entnehmbaren intraokularen Arzneimittelabgabevorrichtung mit kontinuierlicher Freisetzung, vorzugsweise nach einem der vorhergehenden Ansprüche 1 bis 14, Folgendes umfassend:
- Bereitstellen eines länglichen Körpers (3), der einen Polymermatrixkern und eine Polymerbeschichtung umfasst, die den Matrixkern vollständig umgibt, wobei in den Polymermatrixkern mindestens ein therapeutisches Mittel gemischt ist, wobei die zwei Enden des länglichen Körpers nicht versiegelt werden,
- Positionieren des länglichen Körpers in einem Formwerkzeug, das den länglichen Körper zu einem Ring von nahezu 360° formt,
- Hinzufügen mindestens eines Polymers, um die Öse (2) zu bilden, wobei das Polymer außerdem die zwei Enden (4, 5) des länglichen Körpers (3) versiegelt.

## Revendications

1. Dispositif d'administration intraoculaire de médicament à libération prolongée (1), comprenant : un corps allongé (3) comprenant deux extrémités, et un oeillet (2) relié de manière fixe à au moins une extrémité du corps allongé ; dans lequel ledit corps allongé (3) comprend en outre un noyau matriciel polymère sensiblement allongé dans lequel au moins un agent thérapeutique est mélangé, et une enveloppe entourant complètement ledit noyau matriciel, ladite enveloppe comprenant, et étant de préférence, un revêtement polymère ; dans lequel ledit oeillet (2) est conçu pour faciliter le retrait dudit dispositif (1) et dans lequel ledit oeillet (2) est essentiellement exempt d'agent thérapeutique, et dans lequel l'œillet comprend une partie de préhension (8) et une partie de liaison (7), ladite partie de liaison (7) reliant la partie de préhension (8) à au moins une extrémité, de préférence aux deux extrémités, du corps.

2. Dispositif d'administration intraoculaire de médicament à libération prolongée (1) selon la revendication 1, dans lequel la partie de préhension (8) comprend une ouverture (6).

3. Dispositif d'administration de médicament intraoculaire à libération prolongée (1) selon la revendication 1 ou 2, comprenant en outre une section rétrécie (11) comprise dans la partie de liaison (7) ou étant positionnée entre la partie de préhension (8) et la partie de liaison (7), ladite section rétrécie (11) facilitant le pliage dudit dispositif d'administration de médicament (1).

4. Dispositif d'administration intraoculaire de médicament à libération prolongée (1) selon l'une quelconque des revendications précédentes 1 à 3, dans lequel les extrémités (4, 5) du corps allongé (3) sont scellées, et les extrémités scellées (4, 5) sont exempt d'agent thérapeutique, dans lequel au moins une extrémité scellée (4,5) est scellée par l'œillet (2), de préférence les deux extrémités (4,5) sont scellées par l'œillet (2).

5. Dispositif d'administration intraoculaire de médicament à libération prolongée (1) selon l'une quelconque des revendications précédentes 1 à 4, dans lequel l'œillet (2) comprend un ou plusieurs des polymères suivants : éthylène-acétate de vinyle (EVA), poly(diméthylsiloxane) ou PDMS, polyoléfines, polyéthylène, polyuréthane, poly(méthacrylate de méthyle) ou PMMA, et alcool polyvinylique réticulé ou une combinaison de ceux-ci.

6. Dispositif d'administration intraoculaire de médicament à libération prolongée (1) selon la revendication 5, dans lequel le polymère composant l'œillet (2) est compatible avec le polymère inclus dans le corps allongé (3), ledit polymère du corps allongé (3) comprenant : éthylène-acétate de vinyle (EVA), poly(diméthylsiloxane) ou PDMS, polyoléfines, polyéthylène, polyuréthane, poly(méthacrylate de méthyle) ou PMMA, polyuréthane thermoplastique (TPU), et alcool polyvinylique réticulé ou une combinaison de ceux-ci.

7. Dispositif d'administration intraoculaire de médicament à libération prolongée (1) selon l'une quelconque des revendications précédentes 1 à 6, dans lequel l'œillet (2) comprend en outre une jonction (10) reliant la partie de préhension (8) et la partie de liaison (7), dans lequel ladite jonction (10) comprend la section rétrécie (11).

8. Dispositif d'administration intraoculaire de médicament à libération prolongée (1) selon l'une quelconque des revendications 1 à 7, dans lequel la partie de préhension (8) et la partie de liaison (7) sont directement reliées, et dans lequel l'ouverture (6) dans la partie de préhension (8) s'étend en partie dans une section de la partie de liaison (7), définissant ainsi la section rétrécie (11), moyennant quoi ladite section rétrécie (11) a une largeur minimale dans la dimension le long de laquelle la partie de préhension (8) s'étend depuis le corps allongé (3), laquelle largeur minimale de la section rétrécie (11) est inférieure à la largeur minimale dans ladite dimension des sections environnantes de la partie de liaison (7).

9. Dispositif d'administration intraoculaire de médicament à libération prolongée (1) selon l'une quelconque des revendications précédentes 1 à 8, dans lequel ledit dispositif (1) est un anneau à 360° et l'œillet (2) est fixé audit dispositif (1) en deux points de fixation (4, 5), dans lequel les deux points de fixation (4, 5) scellent respectivement les deux extrémités du corps allongé (3) dudit dispositif (1), de préférence, dans lequel l'œillet (2) fait saillie vers le centre de l'anneau.

10. Dispositif d'administration intraoculaire de médicament à libération prolongée (1) selon l'une quelconque des revendications précédentes 1 à 9, dans lequel ledit dispositif (1) est dans une configuration pliée appropriée pour une injection dans l'oeil avec un dispositif d'injection.

11. Dispositif d'administration intraoculaire de médicament à libération prolongée (1) selon l'une quelconque des revendications précédentes 1 à 10, dans lequel l'œillet (2) s'étend depuis le corps allongé (3) sur une distance allant de 1,0 mm à 3,5 mm, de préférence de 1,5 mm à 3,0 mm, plus préférentiellement entre 1,7 mm et 2,7 mm, encore plus préférentiellement entre 2,0 et 2,6 mm.

12. Dispositif d'administration intraoculaire de médicament à libération prolongée (1) selon l'une quelconque des revendications précédentes 1 à 11, dans lequel la dimension de largeur maximale de l'ouverture (6) dans ledit oeillet va de 0,1 mm et 1,0 mm, de préférence de 0,2 mm à 0,6 mm, plus préférentiellement de 0,25 mm à 0,4 mm.

13. Dispositif d'administration intraoculaire de médicament à libération prolongée (1) selon l'une quelconque des revendications précédentes 1 à 12, dans lequel l'ouverture (6) est définie par deux bras qui se rejoignent au niveau de leurs extrémités respectives, dans lequel la largeur minimale des bras est de 0,15 mm.

14. Dispositif d'administration intraoculaire de médicament à libération prolongée (1) selon l'une quelconque des revendications précédentes 1 à 13, dans lequel la dimension transversale de la partie de préhension (8) dans le plan défini par l'ouverture (6) va de 0,5 mm à 1,4 mm, de préférence de 0,6 mm à 0,9 mm, idéalement d'environ 0,7 mm à 0,8 mm.

15. Procédé pour préparer un dispositif d'administration intraoculaire de médicament à libération prolongée amovible, de préférence selon l'une quelconque des revendications précédentes 1 à 14, comprenant :
- la fourniture d'un corps allongé (3) comprenant un noyau de matrice polymère et un revêtement polymère entourant complètement ladite matrice polymère, dans lequel au moins un agent thérapeutique est mélangé dans le noyau de matrice polymère, dans lequel les deux extrémités du corps allongé ne sont pas scellées ;
- le positionnement du corps allongé dans un moule qui forme ledit corps allongé en un anneau de près de 360° ;
- l'ajout d'au moins un polymère pour former l'œillet (2), dans lequel le polymère scelle également les deux extrémités (4, 5) dudit corps allongé (3).
